# EUROPEAN PATENT APPLICATION

(11) **EP 3 228 285 A1**
(43) Date of publication of application: **11.10.2017**
(21) Application number: 17158869.2
(22) Date of filing: 02.03.2017
(51) Int. Cl.: A61F 7/10

(54) **HANDPIECE FOR A CRYOLIPOLYSIS TREATMENT AND APPARATUS FOR A CRYOLIPOLYSIS TREATMENT COMPRISING THEREOF**

(30) Priority: 04.03.2016 IT UA20161373
(71) Applicant: Romiti, Edoardo, 20060 Mediglia (MI) (IT)
(72) Inventor: Romiti, Edoardo, 20060 Mediglia (MI) (IT)
(74) Representative: Fiorentino, Luca

(57) **Abstract**

The invention consists of a handpiece for a cryolipolysis treatment. The handpiece includes an almost dome-shaped wall with an elliptical base which delimits a seat into which can be inserted, at least partly, a part of the human body to be subjected to the treatment of cryolipolysis. The delimiting wall comprises a first opening, to the top of the handpiece, in correspondence of which, the latter is connectable to a suction device to create a depression inside the said seat. The delimiting wall also includes a second opening, at the base of the handpiece, to enable the insertion of the part of the human body, to be subjected to the treatment, in the above seat. The thickness of the delimiting wall is not uniform. More precisely, with reference to the elliptical ring corresponding to a section of the delimiting wall parallel to the second opening, the inner edge of that elliptical crown is more eccentric of the outer edge of the same. The wall of delimitation is therefore thicker in correspondence of the two mutually opposed portions of the same intercepted by the minor axis of that elliptical section. In a first execution of the handpiece of the invention, the delimiting wall includes two closed cavities respectively formed in the two wall portions to greater thickness and containing a thermal gel. In a second execution of the handpiece of the invention, the handpiece is devoid of cavities in the delimiting wall and includes an almost ring shaped band containing thermal gel bags; such band is connected to the delimiting wall so as to surround the handpiece, externally. The invention also relates to an apparatus for cryolipolysis treatment, including the handpiece and a manual vacuum pump connectable to the handpiece.

## Description

### Field of application of the invention

The present invention finds application in the branch of aesthetic medicine for the correction of imperfections caused by fat deposits accumulated in excess in the subcutaneous layer.

More precisely, the present invention relates to the cryolipolysis aesthetic treatment, that consists in not-invasive treatments, by cooling subcutaneous adipose deposits to cause apoptosis of adipocytes and the resulting in biological elimination of the same by organism. The apoptosis of the adipocytes occurs by crystallization as a result of prolonged exposure of the same (e.g. thirty to sixty minutes) to temperatures generally below 0 ° C.

The equipment for cryolipolysis treatments include, generally, an aspirator connected to one or more plastic or glass "bells", substantially rigid, for creating a depression inside the same. The "bells", usually called "hand pieces", are dome-shaped with rectangular or oval base, and are connected to suitable cooling systems capable of cooling the internal environment to the handpiece. The procedure for a cryolipolysis treatment is:
a) apply the handpiece on a single zone of the subject's skin to be subjected to the cryolipolysis treatment;
b) operate the manual vacuum pump to create a depression inside the handpiece, by which the skin and the underlying adipose accumulations are partially sucked into the handpiece;
c) actuate the cooling means to refrigerate the internal environment to the handpiece, and with it the skin and the adipose accumulations therein partially sucked.

The present invention refers to a handpiece for a cryolipolysis treatment and an apparatus for a cryolipolysis treatment comprising the handpiece object of the invention.

### Prior art overview

In the known equipment for cryolipolysis treatments the cooling means consist, usually, in one or more heat pumps applied to the walls of each handpiece. These heat pumps are, for example, Peltier cells, and require a power supply to be added to that of the vacuum system. In addition to that, the placement of a thermoelectric component very close to the human body involves the adoption of considerable security measures which complicate the structure of existing equipment for cryolipolysis treatments.

### Purposes of the invention.

The purpose of the present invention is to overcome the aforesaid drawbacks, indicating a handpiece for a cryolipolysis treatment which is not connected to any electric device nor similar thermoelectric equipment nor electric vacuum pump to result more comfortable for a person undergoing the cryolipolysis treatment.

### Summary of the invention

The present invention is a handpiece for cryolipolysis treatment comprising:
- At least one wall, almost dome-shaped, delimiting a seat in which a part of the human body to be subjected to the cryolipolysis treatment can be at least partially received.
- At least a first opening in said wall at which the handpiece can be connected to pneumofore means which are suitable for creating, inside said seat, a depression with respect to an external environment.
- At least a second opening in said wall for allowing the insertion of said part of the human body into said seat, said second opening having a width in a first direction (defined as "longitudinal" in the following description) greater than the width in a second direction (defined as "transversal" in the following description) arranged orthogonally to said first direction;
wherein, according to the invention, this handpiece comprises at least one thermal gel connected to said wall so as to allow conduction heat exchange between said gel and said wall,
said delimiting wall being at least partially elastically deformable,
said delimiting wall having at least one section (defined as "orthogonal" in the following pages) arranged almost parallel to an edge delimiting said second opening, a thickness in said transversal direction greater than the thickness in said longitudinal direction, said delimiting wall thus comprising two portions of larger thickness mutually opposite along said transversal direction.

As "thermal gel" we intend a material with high specific heat capacity, in fact a material with isobaric specific heat capacity preferably not lower than 2000 J/(Kg·K) and more preferably not lower than 4186 J/(Kg·K). As an example, such gel can be composed by polyacrylamide and/or carboxymethyl cellulose and/or propanediol and/or propylene glycol. Preferably, the thermal gel is composed of water, carboxymethyl cellulose and 1.2 propanediol. This thermal gel, quoted as example, is well known (So we will not continue with further details about it) and offers the advantage to be malleable also at temperatures lower than 0 degrees Centigrades.

Incidentally, it is necessary that the gel and the wall are in contact with each other directly or through the interposition of other materials in order to be possible to have a thermal conduction between the gel and the handpiece wall.

As "width of the second opening in said longitudinal direction" we intend the distance between two points obtained by the intersection of an axis oriented in said longitudinal direction and the edge delimiting said second opening. Incidentally, being the second opening obtained in this delimiting wall, the edge of the second opening is a closed line drawn on such delimiting wall.

As "width of the second opening in said transversal direction" we intend the distance between two points obtained by the intersection of an axis oriented in such transversal direction and the delimiting edge of the second opening.

As "orthogonal section of the handpiece wall" we intend a section of said wall in a direction almost parallel with the delimiting edge of the second opening. Said orthogonal section is then a portion of a plane almost ring shaped and delimited by an internal edge laying on the side of the handpiece wall facing said seat, and an external edge laying on the side of the handpiece wall facing the outside.

As "thickness of the handpiece wall in said transversal direction in correspondence with this orthogonal section" we intend the distance between the above said internal and external edges measured in said orthogonal section along this transversal direction. Being the orthogonal section almost ring shaped, the thicknesses measurable in said transversal direction are two. In case they were not identical, as thickness in said transversal direction we intend the greater of the two thicknesses.

As "thickness of the handpiece wall in such longitudinal direction in correspondence with said orthogonal section" we intend the distance between the above said internal and external edges measured in said orthogonal section along said longitudinal direction. Being the orthogonal section almost ring shaped, the thicknesses measurable in this longitudinal direction are two. In case they were not identical, as thickness in said longitudinal direction we intend the greater of the two thicknesses.

In the handpiece object of the invention, to refrigerate the environment inside of it (the above defined seat) it is enough to refrigerate the thermal gel at a temperature not exceeding the temperature you desire to reach inside the handpiece. The thermal gel works as a thermal buffer and subtracts heat from the internal environment to the handpiece mostly by thermal conduction, allowing in such way the cryolipolysis treatment. It is possible to refrigerate the thermal gel for instance placing the handpiece in a home freezer for a set time before using it for the treatment. The handpiece object of the invention is then not connected to electrical heat pumps or similar thermoelectric equipment like Peltier cells.

Thanks to the reduced thickness of the handpiece wall in said longitudinal direction, this delimiting wall is easily deformable with the result of narrowing the second opening. In particular, after having applied the handpiece in correspondence with the part of human body to be treated with cryolipolysis and after having operated the vacuum devices, the depression inside the seat does not only raise the skin towards the first opening, but it modifies the delimiting edge of the second opening increasing its eccentricity. In this way the skin is introduced in the handpiece both by suction and by the tightening due to the warping of the delimiting edge of the second opening. This results in a better comfort of the subject undergoing the cryolipolysis treatment, especially in the case where the handpiece object of the invention will be used, together with the aforementioned vacuum devices, as a device for home self-treatment. In that case, in fact, the person operating the equipment is at the same time the person undergoing treatment. The higher comfort for the patient/operator is due to both the flexibility of the handpiece object of the invention and the "pinching" effect described above, which allow the patient/operator to manually regulate the depression inside the handpiece and the warping of the same according to his sensations.

Further innovating technical features of the present invention are described in the dependent claims.

The above described delimiting wall is preferably dome shaped with a almost elliptical base. In such case, the delimiting edge of the second opening is almost elliptical, with the ellipse longer axis corresponding with said longitudinal direction and the ellipse shorter axis corresponding with said transversal direction.

If the wall of the handpiece is dome shaped with an almost elliptical base, the above mentioned orthogonal section is almost shaped as an elliptical crown, i.e. a crown which, instead of being round, is delimited by two coplanar ellipses with longer and shorter axis respectively superimposed. Such coplanar ellipses correspond respectively to the above mentioned inner edge and outer edge of the ring section of the handpiece wall.

If the orthogonal section is almost conformed as an elliptical crown, the thickness of the handpiece wall in said transversal direction corresponds to the thickness of the handpiece wall measured along the shorter axis of the two above mentioned coplanar ellipses, and the thickness of the handpiece wall in said longitudinal direction corresponds to the thickness of the handpiece wall measured along the longer axis of the two above mentioned coplanar ellipses.

In particular, if the orthogonal section is almost conformed as an elliptical crown, being the thickness of the handpiece wall not uniform, the two above said ellipses have different eccentricity. More precisely, having the handpiece wall, in correspondence with said orthogonal section, a thickness in said transversal direction greater than the thickness in said longitudinal direction, the inner edge of the elliptical crown is more eccentric compared with the outer edge of the same.

As an advantage, being the handpiece object of the invention dome shaped with almost elliptical base, the absence of corners in the delimiting edge of the second opening reduces the inconvenience caused to who is undergoing the cryolipolysis treatment compared with the use of the known dome shaped handpieces with an almost rectangular base. The above mentioned elliptical shape assures also a better uniformity of the exposition to the thermal gel of the portion of skin sucked into the handpiece during a cryolipolysis treatment.

Another object of the invention is an apparatus for a cryolipolysis treatment comprising:
- At least one handpiece according to the present invention
- pneumofore means, which can be connected to the handpiece at said first opening and suitable for creating, inside said seat, a depression with respect to an external environment.

### Brief description of figures

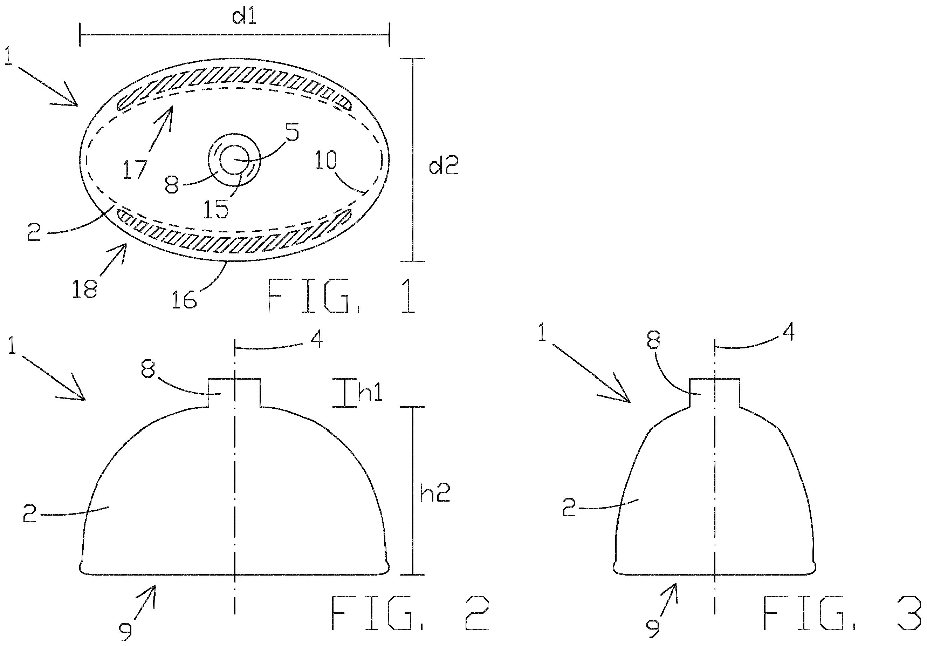

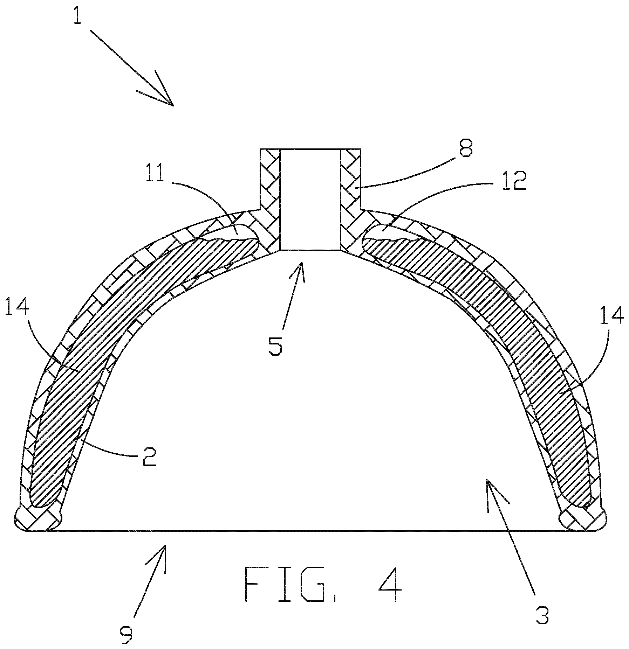

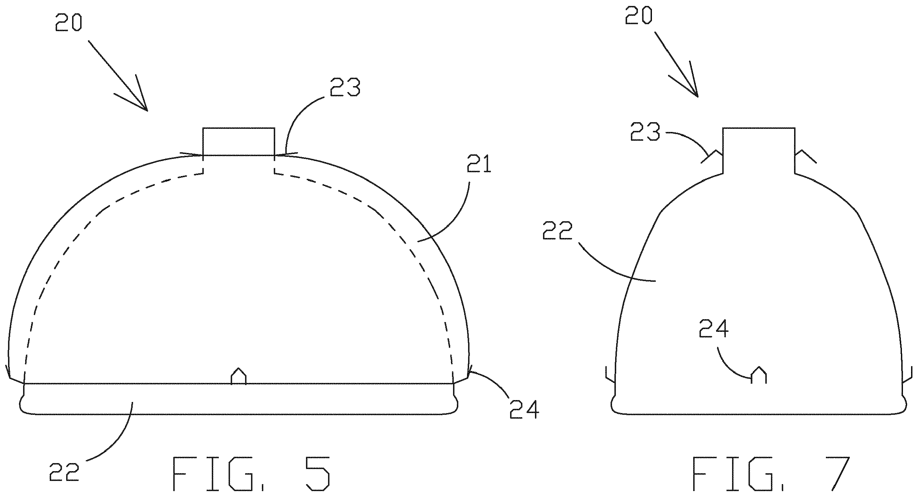

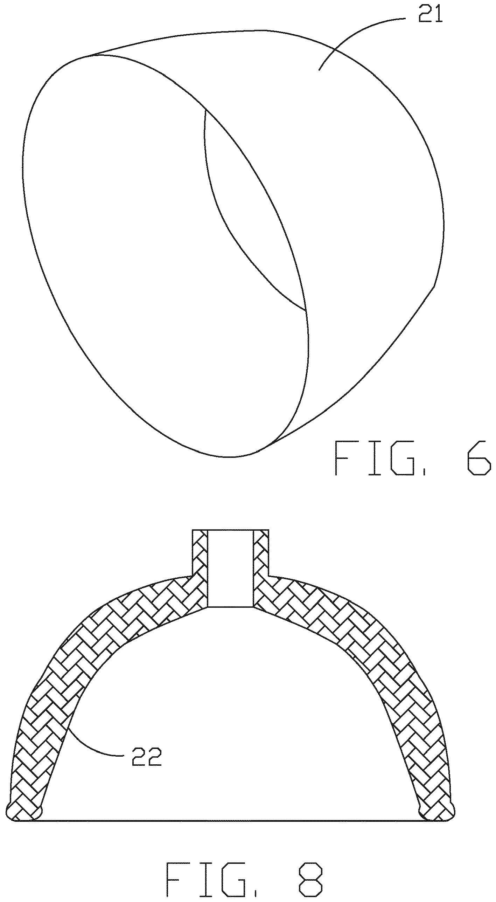

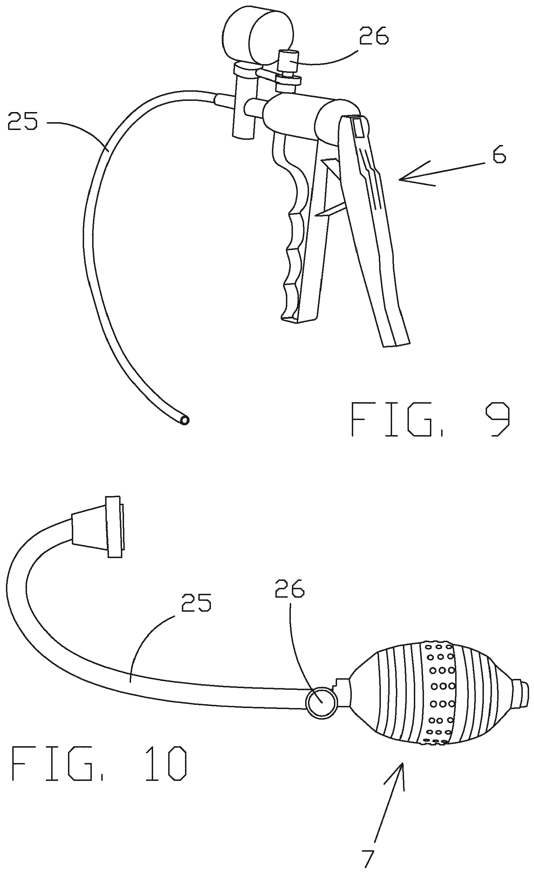

Further scopes and advantages of the present invention will be clarified by the detailed description which gives an example of a preferred embodiment thereof and by the attached drawings, provided with an explicative but not limitative purpose, in which:
◆ **figure 1** shows, in flat top view, one handpiece according to the present invention;
◆ **figure 2** shows the figure 1 handpiece in flat front view;
◆ **figure 3** shows the figure 1 handpiece in flat side view;
◆ **figure 4** shows the figure 1 handpiece in schematic cross section ◆ **figure 5** shows, in flat front view, a variation of the handpiece in figure 1;
◆ **figure 6** shows, in perspective view, one first component, more external, of the handpiece in figure 5;
◆ **figure 7** shows, in flat side view, a second component, more internal, of the handpiece in figure 5;
◆ **figure 8** shows the component in figure 5 in schematic cross section;
◆ **figures 9 & 10** show, in perspective view, two aspiration devices connectable with the handpieces of figures 1 & 5.

### Detailed description of preferred embodiments of the invention

In the continuation of the present description one figure can be illustrated also with reference to elements not expressly indicated in that figure but in other figures. The ratio and proportions of the different elements do not need to correspond to the real ones.

**Figures 1 to 4** show a handpiece 1 usable for a cryolipolysis treatment. Handpiece 1, in its general configuration, substantially looks like one hemisphere compressed parallely to the base. In the preferred embodiment described here, handpiece 1 resembles one of the two halves which are obtained by cutting a rugby ball in parallel with its longitudinal axis. More precisely, handpiece 1 is almost dome shaped and comprises a wall 2, preferably concave, which delimits a seat 3 in which a part of the human body (not visible in figures) to be subjected to the cryolipolysis treatment can be at least partially received. Handpiece 1 is preferably symmetric in respect of an axis 4 orthogonal to the plane of the sheet in figure 1, and laying on the plane of the sheet in figures 2,3 & 4. Incidentally, axis 4 is placed orthogonally to the plane along which the above mentioned rugby ball is divided in two halves. As seen in figure 1, the wall 2 is dome shaped with a substantially elliptical base.

Wall 2 comprises, at the top of handpiece 1, one first opening 5 through which it is possible to connect handpiece 1 to a vacuum device 6 or 7 (respectively shown in figure 9 and 10) to create a depression inside the seat 3. The opening 5 is preferably circular with an internal diameter for example included between 0.5 and 2 cm. In order to facilitate the connection of vacuum device 6 or 7 to handpiece 1, the latter comprises at the opening 5 a bushing 8, preferably cylindrical, which extends towards the outside of handpiece 1 (in the opposite direction of seat 3) from circular edge 15 which delimits opening 5. This bushing has a length h1 preferably between 0.5 and 2 cm.

Wall 2 comprises, at the base of handpiece 1, a second opening 9 through the part of the human body to be subjected to the cryolipolysis treatment can be received into seat 3. Opening 9 is not circular. More precisely, it is wider in longitudinal direction than in transversal direction. In particular opening 9 is preferably elliptical, with major axis corresponding to the above said longitudinal direction and minor axis corresponding to the above side transversal direction. Being handpiece 1 symmetric in respect of axis 4, circular edge 15 which delimits opening 5 and elliptical edge 10 which delimits opening 9 are orthogonal to axis 4 and coaxial to each other. The distance h2 between openings 5 and 9 (corresponding to the height of wall 2) is preferably comprised between 7 and 10 cm. Incidentally, as shown in figure 1, edges 5 and 15 laying in correspondence to the internal side of wall 2, i.e. corresponding to the side of the wall 2 facing seat 3.

Wall 2 and bushing 8 are preferably manufactured in polymeric material and are at least partially, elastically deformable.

As visible in figure 1, the thickness of wall 2 is not uniform. More precisely, with reference to a ring section of wall 2 placed orthogonally to axis 4 (and parallel to edges 10 and 15), wall 2 is thicker in transversal direction (i.e. along the above said minor axis) than in longitudinal direction (i.e. along above said the major axis). Preferably, the thickness of wall 2 in transversal direction is not more than three times (3x) the thickness in longitudinal direction. Since wall 2 is preferably dome shaped with almost elliptical base, the above mentioned ring section of wall 2 has the shape of an elliptical crown, i.e. a crown which is not circular but delimited by two coplanar ellipses having the major and minor axis respectively superimposed. Such coplanar ellipses correspond then to inner and outer edges of such elliptical crown, and are respectively laying in correspondence with the inner side (facing seat 3) and the outside side (facing the surrounding) of wall 2. With reference to the section of wall 2 in correspondence of opening 9, such inner edge coincides with edge 10 of opening 9, and said outer edge coincides with edge 16 of wall 2 as shown in figure 1. Being wall 2 thicker in transversal than in longitudinal direction, the inner edge of the elliptical crown is more eccentric than the outer one. Preferably the eccentricity of said inner edge is not more than double of said external one. Wall 2 is then thicker in correspondence of two portions of the same 17 and 18, mutually opposed and crossed by the minor axis of said elliptical crown. Incidentally, portions 17 and 18 are opposed along such transversal direction.

With reference to the elliptical outer edge 16, the longer axis of the ellipse (corresponding to the longitudinal direction) has a length d1 preferably comprises between 6 and 16 cm, and the shorter axis of the ellipse (corresponding to the transversal direction) has a length d2 preferably comprises between 4 and 10 cm.

**Figure 4** shows handpiece 1 in cross section, i.e. parallel to the shorter axis on the above mentioned elliptical crown. As shown in figure 4, wall 2 includes several closed cavities 11 and 12 filled with a thermal gel 14, preferably a gel based on polyacrylamide or on water, carboxymethyl cellulose and 1.2 propanediol. Gel 14 is direct contact with wall 2. In this way between gel 14 and wall 2 there can be a thermal exchange by conduction. Cavities 11 and 12, as example being in number of 2, are realized respectively in correspondence of portions 17 and 18 of greater thickness of wall 2. In particular, wall 2 includes at least one of such cavities in correspondence of each of said portions 17 and 18. Preferably, cavities 11 and 12 extend vertically, in figure 4, between openings 5 and 9, and longitudinally, in figure 1, almost for the whole length of sections 17 and 18, in order to surround, at least partially, seat 3. The cross section of cavities 11 and 12 in this way has elongated shape. Handpiece 1 includes an amount of gel 14 preferably comprised between 250 and 500 grams.

**Figure 5** shows one handpiece 20 which is different from handpiece 1 for the fact that the thermal gel, instead of being enclosed in cavities realized into wall 22 (corresponding to wall 2 of handpiece 1), is received in a specific containment structure, i.e. a band 21, positioned on wall 22 in such way to surround it externally (i.e. on the opposite side of seat 3 with respect to wall 22). In particular, band 21 extends from the circular edge 15 of opening 5 until the proximity of elliptical edge 10 of opening 9, and, as shown in **figure 6****,** it is preferably shaped as ring shaped band with almost the same curvature of wall 22. More precisely, band 21 includes one pair of mutually opposed thin walls (the internal dotted ones in figure 5) between which is enclosed the thermal gel. Band 21 is connected to wall 22 in order to adhere to its external surface at least partially, to allow a thermal exchange by conduction between the thermal gel and wall 22. The walls of band 21 are preferably realized in polymeric material and, as example, have a thickness between 1 and 3 millimeters. Band 21 encloses an amount of thermal gel preferably coprised between 250 and 500 grams.

In reference with **figure 7** it can be noted that the connection between band 21 and wall 22 is realized through a number of hooks 23 and 24 integrally connected to wall 22 and to bushing 8 in correspondence of their external surface. Hooks 23 and 24 are placed in proximity of opening 5 and opening 9. Hooks 23, present as example in number of 4, are connected to bushing 8 and are placed along a circle preferably parallel to round edge 15 which delimits opening 5. Hooks 24, also present as example in number of 4, are connected to wall 22 in proximity of elliptical edge 16, and are place along an ellipses preferably parallel to it. As shown in figure 5, band 21 is wedged between hooks 23 and 24 in a way which allows removal. In addition, or in alternative to the presence of hooks 23 and 24, band 21 and wall 22 can be connected to each other with Velcro (not shown in figures) interposed between them.

In an alternative form of manufacturing of handpiece 20, not shown in figures, band 21 is at least partially elastic and can be hooked to hooks 23 and 24 of the bushing 8 and the wall 22.

**Figure 8****,** like figure 4, shows handpiece 20 in cross section. Wall 22 is shaped like wall 2 and is different from it for the absence of cavities 11 and 12. The characteristics of elliptical shape of the ring section and of grater thickness in transversal direction as described for wall 2 are valid also for wall 22.

**Figures 9 & 10** respectively show one manual aspirator 6 of pneumatic cylinder type and a manual aspirator 7 of single ball type, which can be connected to bushing 8 of handpieces 1 and 20 through a flexible pipe 25. The connection between aspirator 6 or 7 and handpiece 1 or 20 realizes a piece of equipment for applying cryolipolysis treatments. As previously stated, aspirators 6 and 7 are in fact connectable to handpieces 1 and 20 in order to create a depression inside seat 3 so to suck into it the body part to be subjected to the cryolipolysis treatment.

Aspirators 6 and 7 are preferably equipped with a release valve (not shown in pictures) and a safety valve 26 to avoid that absolute pressure inside seat 3 reaches values below a predetermined limit value, preferably equal to 80 kPa.

In order to implement a cryolipolysis treatment by an apparatus comprising handpiece 1 and aspirator 6 or 7 it is possible to carry out the following steps:
a) place handpiece 1 in an environment with a temperature preferably not higher than-18 degrees Celsius (for instance inside a domestic freezer) for a period of time preferably not shorter than 60 minutes;
b) connect handpiece 1 to aspirator 6 or 7;
c) apply handpiece 1, in correspondence with opening 9, on the skin of the body part to be subjected to cryolipolysis;
d) operate aspirator 6 or 7 in order to reach inside seat 3 an absolute pressure lower than the ambient pressure (surrounding area pressure), and preferably lower than 80 kPa. This will cause the skin to be lifted into seat 3;
e) wait for the elapsing of a time preferably longer than 45 minutes;
f) Operate the release valve of aspirator 6 or 7 and remove handpiece 1.

In order to implement a cryolipolysis treatment by an apparatus comprising handpiece 20 and aspirator 6 or 7 it is possible to carry out the following steps:
a) place band 21 in an environment with a temperature preferably not higher than - 18 degrees Celsius (for instance inside a domestic freezer) for a period of time preferably not shorter than 60 minutes;
b) connect band 21 to wall 22 and connect handpiece 20 to aspirator 6 or 7
c) apply handpiece 20, in correspondence with opening 9, on the skin of the body part to be subjected to cryolipolysis;
d) operate aspirator 6 or 7 in order to reach inside seat 3 an absolute pressure lower than the ambient pressure (surrounding area pressure), and preferably lower than 80 kPa. This will cause the skin to be lifted into seat 3;
e) wait for the elapsing of a time preferably longer than 45 minutes;
f) Operate the release valve of aspirator 6 or 7 and remove handpiece 20.

In both these ways the thermal gel subtracts heat from the cavity of the handpiece, cooling it down and in this way refrigerating also the portion of human body held into the cavity by the depression created into it.

On the base of the provided description as an example of preferred embodiment, of course some changes can be introduced by the skilled in the art without changing the scope of the invention as defined by the following claims.

## Claims

1. A handpiece (1, 20) for a cryolipolysis treatment, comprising:
• at least one wall (2, 22) delimiting a seat (3) in which a part of the human body to be subjected to the cryolipolysis treatment can be at least partially received, said wall (2, 22) being dome-shaped;
• at least a first opening (5) in said wall (2, 22) at which said handpiece (1, 20) can be connected to pneumofore means (6, 7) which are suitable for creating, inside said seat (3), a depression with respect to an external environment;
• at least a second opening (9) in said wall (2, 22) for allowing the insertion of said part of the human body into said seat (3), said second opening (9) having a width in a first direction greater than the width in a second direction arranged orthogonally to said first direction;
said handpiece (1, 20) being **characterized in that** it comprises at least one thermal gel (14) connected to said wall (2, 22) so as to allow a conduction heat exchange between said gel (14) and said wall (2, 22),
said wall (2, 22) being at least partially elastically deformable,
said wall (2, 22) having, at least one section arranged almost parallel to an edge (10) delimiting said second opening (9), a thickness in said first direction greater than the thickness in said second direction, said wall (2, 22) thus comprising two portions (17, 18) of larger thickness mutually opposite along said second direction.

2. A handpiece according to claim 1, **characterized in that** said wall (2, 22) is dome-shaped with a substantially elliptical base.

3. A handpiece (1) according to claim 1 or 2, **characterized in that** said wall (2) includes at least one closed cavity (11, 12) in which said thermal gel (14) is accommodated.

4. A handpiece (1) according to claim 1 or 3, **characterized in that** said wall (2) includes at least one said cavity (11, 12) at each said portion (17, 18) of greater thickness.

5. A handpiece (20) according to claim 1 or 2, **characterized in that** it comprises:
• at least one containment structure (21) of said thermal gel (14);
• means (23, 24) for connecting said containment structure (21) to said wall (22) outside said seat (3).

6. A handpiece (20) according to claim 5, **characterized in that** said containment structure (21) is shaped as a band connectable to said wall (22) so as to surround at least partially the latter.

7. A handpiece (20) according to claim 5 or 6, **characterized in that** said connecting means (23, 24) comprise at least two hooks (23, 24) integrally connected to said wall (22) outside said seat (3), said containment structure (21) being capable of being wedged between said hooks (23, 24).

8. A handpiece (20) according to claim 7, **characterized in that** said hooks (23, 24) are connected to said wall (2) so as to lie close to said first and second openings (5, 9), respectively.

9. A handpiece according to claim 5 or 6, **characterized in that** said connecting means comprise Velcro interposed between said containment structure (21) and said wall (22).

10. An apparatus for a cryolipolysis treatment, comprising:
• at least one handpiece (20) according to one of the preceding claims;
• pneumofore means (6, 7) which can be connected to said handpiece (1, 20) at said first opening (5) and suitable for creating, inside said seat (3), a depression with respect to an external environment.
